# EUROPEAN PATENT APPLICATION

(11) **EP 4 700 011 A1**
(43) Date of publication of application: **25.02.2026**
(21) Application number: 24792416.0
(22) Date of filing: 19.03.2024
(51) Int. Cl.: C07C 53/126, G01N 27/62, G01N 30/72, G01N 30/88

(54) **3,5,5-TRIMETHYLHEXANOIC ACID COMPOSITION AND METHOD FOR IMPROVING STORAGE STABILITY THEREOF**

(30) Priority: 21.04.2023 JP 2023070015
(71) Applicant: KH Neochem Co., Ltd., Tokyo 103-0022 (JP)
(72) Inventor: OHGATA, Yusuke, Ichihara-shi, Chiba 290-8560 (JP); KITABATAKE, Kouji, Ichihara-shi, Chiba 290-8560 (JP)
(74) Representative: Maiwald GmbH
(86) International application number: PCT/JP2024/010656
(87) International publication number: WO 2024/219140

(57) **Abstract**

A 3,5,5-trimethylhexanoic acid composition containing 3,5,5-trimethylhexanoic acid and t-butyl alcohol in which the t-butyl alcohol concentration in terms of toluene-d8 measured by gas chromatography-olfactometry/mass spectrometry after heating in an air atmosphere at 80°C for four weeks under the conditions below exceeds 0 ppb by volume and is 400 ppb by volume or less is provided. The composition has low reproductive toxicity even after long-term storage:
(Measurement Conditions)
· the 3,5,5-trimethylhexanoic acid composition is concentrated, and
· the concentrated sample is measured by gas chromatography-olfactometry using a column using dimethylpolysiloxane as a stationary phase, and the structure of a measured component is analyzed with a mass spectrometry detector.

## Description

### Technical Field

The technique of the present disclosure relates to a 3,5,5-trimethylhexanoic acid composition and a method for improving the storage stability thereof.

### Background Art

It is known that 3,5,5-trimethylhexanoic acid can be synthesized by oxidation of 3,5,5-trimethylhexanal, which is a precursor aldehyde (for example, see PTL 1). Moreover, a method for synthesizing 3,5,5-trimethylhexanoic acid by oxidation of an alcohol is also known (for example, see PTL 2).

3,5,5-Trimethylhexanoic acid is used as a raw material of a cosmetic product, a raw material of a refrigeration oil and a raw material of a metal working oil or the like. A cosmetic product, a refrigeration oil and a metal working oil may cause exposure, although slightly, due to the characteristics of the applications thereof, and thus the raw materials thereof are required to have low toxicity.

t-BuOH, which is reproductively toxic, is produced as a by-product during the production process of 3,5,5-trimethylhexanoic acid, but it has been believed that the amount thereof can be already reduced sufficiently by the conventional purification method.

### Citation List

### Patent Literature

PTL 1: WO 2022/118917
PTL 2: CN112409144

### Summary of Invention

### Technical Problem

However, it was newly found that the amount of t-BuOH increases when 3,5,5-trimethylhexanoic acid is stored for a long time.

The technique of the present disclosure was made under the above circumstances. A problem of the technique of the present disclosure is to provide a 3,5,5-trimethylhexanoic acid composition which has low reproductive toxicity even after long-term storage and a method for improving the storage stability thereof, and an aim thereof is to solve the problem.

### Solution to Problem

<1> A 3,5,5-trimethylhexanoic acid composition containing 3,5,5-trimethylhexanoic acid and t-butyl alcohol,
   in which the t-butyl alcohol concentration in terms of toluene-d8 measured by gas chromatography-olfactometry/mass spectrometry after heating in an air atmosphere at 80°C for four weeks under the conditions below exceeds 0 ppb by volume and is 400 ppb by volume or less:
   (Measurement Conditions)
   · the 3,5,5-trimethylhexanoic acid composition in an amount of 5.0 g is filled into a sealable 500-mL container and left still at 30°C for 20 minutes or more. Then, 200 mL of the gas phase is sucked, and the residue after removing H₂O, N₂, O₂ and CO₂ is concentrated with a concentrator, and
   · the concentrated sample is measured by gas chromatography-olfactometry under the conditions below, and the structure of a measured component is analyzed with a mass spectrometry detector:
      Analysis column: a column using dimethylpolysiloxane as a stationary phase; a length of 60 m × an inner diameter of 320 µm × a film thickness of 1 µm
      Heating program: After maintaining at 35°C for two minutes, the temperature is increased at 10°C/minute, and the temperature is maintained for seven minutes and 30 seconds after the temperature reaches 240°C.
      Carrier gas: helium
      The measurement conditions of the mass spectrometry detector are as follows.
      Ionization mode: EI
      Measurement type: scan
      Ion source temperature: 250°C
      Quadrupole temperature: 150°C
      Electron energy: 70.0 eV
      Initial mass of scanning: 30
      Final mass of scanning: 400.
<2> The 3,5,5-trimethylhexanoic acid composition according to <1> which is used for a cosmetic product.
<3> The 3,5,5-trimethylhexanoic acid composition according to <1> which is used for a refrigeration oil.
<4> A method for improving storage stability of a 3,5,5-trimethylhexanoic acid composition containing 3,5,5-trimethylhexanoic acid and t-butyl alcohol,
   in which the t-butyl alcohol concentration in terms of toluene-d8 measured by gas chromatography-olfactometry/mass spectrometry under the conditions below is adjusted to exceed 0 ppb by volume and to be 100 ppb by volume or less:
   (Measurement Conditions)
   · the 3,5,5-trimethylhexanoic acid composition in an amount of 5.0 g is filled into a sealable 500-mL container and left still at 30°C for 20 minutes or more. Then, 200 mL of the gas phase is sucked, and the residue after removing H₂O, N₂, O₂ and CO₂ is concentrated with a concentrator, and
   · the concentrated sample is measured by gas chromatography-olfactometry under the conditions below, and the structure of a measured component is analyzed with a mass spectrometry detector:
      Analysis column: a column using dimethylpolysiloxane as a stationary phase; a length of 60 m × an inner diameter of 320 µm × a film thickness of 1 µm
      Heating program: After maintaining at 35°C for two minutes, the temperature is increased at 10°C/minute, and the temperature is maintained for seven minutes and 30 seconds after the temperature reaches 240°C.
      Carrier gas: helium
      The measurement conditions of the mass spectrometry detector are as follows.
      Ionization mode: EI
      Measurement type: scan
      Ion source temperature: 250°C
      Quadrupole temperature: 150°C
      Electron energy: 70.0 eV
      Initial mass of scanning: 30
      Final mass of scanning: 400.
<5> The method for improving storage stability of a 3,5,5-trimethylhexanoic acid composition according to <4>,
   which is a method for improving the storage stability of the 3,5,5-trimethylhexanoic acid composition containing 3,5,5-trimethylhexanoic acid and t-butyl alcohol,
   in which the t-butyl alcohol concentration in terms of toluene-d8 measured by gas chromatography-olfactometry/mass spectrometry after heating in an air atmosphere at 80°C for four weeks under the conditions is adjusted to exceed 0 ppb by volume and to be 400 ppb by volume or less by adjusting the t-butyl alcohol concentration in terms of toluene-d8 measured by gas chromatography-olfactometry/mass spectrometry under the conditions to exceed 0 ppb by volume and to be 100 ppb by volume or less.

### Advantageous Effects of Invention

According to the technique of the present disclosure, a technique related to a 3,5,5-trimethylhexanoic acid composition which has low reproductive toxicity even after long-term storage and a method for improving the storage stability thereof can be provided.

### Description of Embodiments

Any upper limit value and any lower limit value of the numerical ranges described in this description can be combined. For example, when "A to B" and "C to D" are described as numerical ranges, the numerical ranges of "A to D" and "C to B" are also included in the scope of the present disclosure.

In addition, the numerical range "a lower limit value to an upper limit value" described in this description means the lower limit value or more and the upper limit value or less, unless otherwise specified.

### <3,5,5-Trimethylhexanoic Acid Composition>

The 3,5,5-trimethylhexanoic acid composition according to the embodiment is a 3,5,5-trimethylhexanoic acid composition containing 3,5,5-trimethylhexanoic acid and t-butyl alcohol, and the t-butyl alcohol concentration in terms of toluene-d8 measured by gas chromatography-olfactometry/mass spectrometry after heating in an air atmosphere at 80°C for four weeks under the conditions below exceeds 0 ppb by volume and is 400 ppb by volume or less.

### (Measurement Conditions)

· The 3,5,5-trimethylhexanoic acid composition in an amount of 5.0 g is filled into a sealable 500-mL container and left still at 30°C for 20 minutes or more. Then, 200 mL of the gas phase is sucked, and the residue after removing H₂O, N₂, O₂ and CO₂ is concentrated with a concentrator.
· The concentrated sample is measured by gas chromatography-olfactometry under the conditions below, and the structure of a measured component is analyzed with a mass spectrometry detector.
   Analysis column: a column using dimethylpolysiloxane as a stationary phase; a length of 60 m × an inner diameter of 320 µm × a film thickness of 1 µm
   Heating program: After maintaining at 35°C for two minutes, the temperature is increased at 10°C/minute, and the temperature is maintained for seven minutes and 30 seconds after the temperature reaches 240°C.
   Carrier gas: helium
   The measurement conditions of the mass spectrometry detector are as follows.
   Ionization mode: EI
   Measurement type: scan
   Ion source temperature: 250°C
   Quadrupole temperature: 150°C
   Electron energy: 70.0 eV
   Initial mass of scanning: 30
   Final mass of scanning: 400

Regarding the 3,5,5-trimethylhexanoic acid composition according to the embodiment, "after heating in an air atmosphere at 80°C for four weeks" means accelerated conditions for creating a state in which the 3,5,5-trimethylhexanoic acid composition is stored for a long time.

As described above, it has been believed that t-BuOH which is produced as a by-product during the production of 3,5,5-trimethylhexanoic acid can be reduced sufficiently by a conventional purification method such as distillation, and in fact, t-BuOH has not been observed at the detection level by general gas chromatography used for detecting impurities.

However, it was newly found that the amount of t-BuOH increases when 3,5,5-trimethylhexanoic acid is stored for a long time.

As a result of extensive examination on the increase in t-BuOH, it was found through gas chromatography-olfactometry with a higher sensitivity that a very small amount of t-butyl alcohol (t-BuOH) exists in a 3,5,5-trimethylhexanoic acid composition immediately after the production.

In the present invention, the novel problem, namely the existence of a very small amount of t-BuOH in the 3,5,5-trimethylhexanoic acid composition immediately after the production and the increase in t-BuOH after long-term storage of the 3,5,5-trimethylhexanoic acid composition, was found, and the problem is solved by forming the 3,5,5-trimethylhexanoic acid composition with the above configuration.

### [Accelerated Conditions]

The 3,5,5-trimethylhexanoic acid composition according to the embodiment is a 3,5,5-trimethylhexanoic acid composition obtained by heating a 3,5,5-trimethylhexanoic acid composition immediately after production in an air atmosphere at 80°C for four weeks.

The method for producing the 3,5,5-trimethylhexanoic acid composition will be described below.

### [Gas Chromatography-Olfactometry/Mass Spectrometry]

The gas chromatography-olfactometry/mass spectrometry is conducted by concentrating the sample by the method below and then analyzing a component of the concentrated sample by gas chromatography-olfactometry while analyzing the structure of the component with a mass spectrometry detector.

### (Concentration of Sample)

The 3,5,5-trimethylhexanoic acid composition is a liquid at atmospheric pressure (0.1 MPa) at room temperature (25°C). The 3,5,5-trimethylhexanoic acid composition in an amount of 5.0 g is filled into a sealable 500-mL container and left still at 30°C for 20 minutes or more. Then, 200 mL of the gas phase is sucked, and the residue after removing H₂O, N₂, O₂ and CO₂ is concentrated with a concentrator.

The concentrator may be a stand-alone type or may be connected to a gas chromatography-olfactometry/mass spectrometry detector.

As the concentrator, for example, "Entech7200 automated concentrator" manufactured by ENTECH INSTRUMENTS can be used.

The concentration conditions are as follows.

Internal standard substance: 100 mL (standard toluene-d8 gas: concentration of 10 ppb by volume, nitrogen as a dilution gas, Sumitomo Seika Chemicals Company, Limited.)

### Concentration method: CTD mode (Cold Trap Dehydration)

· Temperature condition of Dehydration Module 1 (Empty Trap: a ceramic-coated trap without adsorbent filling): Trap Temp. -40°C, Desorption Temp. 0°C
· Temperature condition of Cold Tenax (registered trademark) Module 2 (Tenax TA Trap: a ceramic-coated trap filled with Tenax TA as an adsorbent): Trap Temp. -30°C, Desorption Temp. 200°C
· Temperature condition of Focusing Module 3 (Cryo focusing): Trap Temp. -165°C, Desorption Temp. 100°C
· Sample flow rate: 50 mL/min
· He Flush Volume: 75 mL
· M1 to M2 Volume: 40 mL (100 mL/min)
· M2 to M3 Time: 3.0 min
· Injection time: 0.3 min

Here, Tenax TA is low polarity porous polymer beads using 2,6-diphenyl-p-phenylene oxide as a base.

M1, M2 and M3 correspond to Modules 1, 2 and 3, respectively, and "M1 to M2" and "M2 to M3" show the conditions of the sample flows between the Modules. The following procedures are conducted in the respective Modules.
M1: removal of water, air and the like
M2: removal of VOC by causing CO₂ to pass through
M3: focusing of the sample

### (Gas Chromatography-Olfactometry)

As the gas chromatography-olfactometry (GC-olfactometry) apparatus, for example, "Agilent 7890B gas chromatography system" manufactured by Agilent Technologies can be used.

The measurement conditions of the GC-olfactometry are as follows.
Analysis column: DB-1 manufactured by Agilent Technologies (a column using dimethylpolysiloxane as a stationary phase; a length of 60 m × an inner diameter of 320 µm × a film thickness of 1 µm)
Heating program: After maintaining at 35°C for two minutes, the temperature is increased at 10°C/minute, and the temperature is maintained for seven minutes and 30 seconds after the temperature reaches 240°C.
Carrier gas: helium
Control mode: constant pressure (153.09 kPa)

The concentrated sample is separated by a capillary column and then sent to ODP3 (sniffing port) and a mass spectrometry detector (for example, MSD5977B below) at a ratio of 1/1.

### (Mass Spectrometry Detector)

As the mass spectrometry detector (MSD), for example, "Agilent 5977B MSD" manufactured by Agilent Technologies can be used.

The measurement conditions of the MSD are as follows.
Ionization mode: EI
Measurement type: scan
Ion source temperature: 250°C
Quadrupole temperature: 150°C
Electron energy: 70.0 eV
Initial mass of scanning: 30
Final mass of scanning: 400
Calibration curve: created using standard toluene-d8 gas manufactured by Sumitomo Seika Chemicals Company, Limited. (concentration: 10 ppb by volume, dilution gas: nitrogen).

For the data analysis, an extracted ion chromatogram (EIC) is used, and the EIC peak area of t-butyl alcohol (EIC: m/z 59.000) that appears in the relative retention time of 0.48 to 0.56 is observed, where the relative retention time of toluene-d8 is regarded as 1.0.

The t-butyl alcohol concentration in terms of toluene-d8 of the 3,5,5-trimethylhexanoic acid vapor is calculated using an equation derived from the calibration curve, "concentration in terms of toluene-d8 (ppb by volume) = 3.94 × 10⁻⁶ × EIC peak area", where the sensitivity of the EIC peak of t-butyl alcohol and the sensitivity of the EIC peak of toluene-d8 are supposed to be equal.

### [t-Butyl Alcohol Concentration in Terms of Toluene-d8]

The t-butyl alcohol concentration in terms of toluene-d8 of the 3,5,5-trimethylhexanoic acid composition according to the embodiment measured by the GC-olfactometry/MSD is preferably 370 ppb by volume or less, more preferably 340 ppb by volume or less, further preferably 200 ppb by volume or less.

### <Method for Producing 3,5,5-Trimethylhexanoic Acid Composition>

The method for producing a 3,5,5-trimethylhexanoic acid composition according to the embodiment is not particularly restricted but preferably has a synthesis step of synthesizing crude 3,5,5-trimethylhexanoic acid and a purification step of purifying the crude 3,5,5-trimethylhexanoic acid.

The purification step preferably includes a water washing step of washing the crude 3,5,5-trimethylhexanoic acid with water and a bubbling step of bubbling the 3,5,5-trimethylhexanoic acid obtained in the water washing step with an inert gas.

### [Synthesis Step]

The crude 3,5,5-trimethylhexanoic acid may be synthesized by a known synthesis method.

For example, the crude 3,5,5-trimethylhexanoic acid can be synthesized by the method described in WO2022/118917. Specifically, for example, 3,5,5-trimethylhexanal is synthesized by hydroformylation reaction of diisobutylene and oxo gas, and crude 3,5,5-trimethylhexanoic acid is synthesized by subsequent oxidation reaction.

### [Purification Step]

The purification step includes a water washing step of washing the crude 3,5,5-trimethylhexanoic acid with water and a bubbling step of bubbling the 3,5,5-trimethylhexanoic acid obtained in the water washing step with an inert gas.

The purification step may further include a distillation step and the like. The distillation step is preferably conducted before the water washing step.

### (Distillation Step)

The distillation step is a step of removing a low-boiling-point component having a lower boiling point than the boiling point of 3,5,5-trimethylhexanoic acid (120°C/13 mmHg).

Specifically, for example, a fraction without high-boiling-point components is put into a three-neck flask having a reflux condenser and a thermometer and distilled at a reduced pressure of 25 kPa, and the fraction of the temperature at the top of the tower of 180 to 200°C is collected to obtain a fraction without low-boiling-point components.

### (Water Washing Step)

The water washing step is a step of washing the crude 3,5,5-trimethylhexanoic acid with water.

The water washing step is a step of mixing the crude 3,5,5-trimethylhexanoic acid obtained in the synthesis step and water and, after phase separation into an aqueous layer and an organic layer, obtaining the organic layer containing 3,5,5-trimethylhexanoic acid.

The water washing step is preferably conducted after the distillation step.

The amount of the water used is preferably 10 to 300 parts by mass, more preferably 20 to 200 parts by mass, based on 100 parts by mass of the crude 3,5,5-trimethylhexanoic acid in view of the efficiency of extraction. When water is mixed with the crude 3,5,5-trimethylhexanoic acid, the temperature is not particularly limited but is preferably a temperature between 5 to 80°C, more preferably a temperature between 10 to 50°C, further preferably a temperature between 10 to 30°C, in view of the efficiency of extraction.

The crude 3,5,5-trimethylhexanoic acid and water can be mixed, for example, by a batch process, a continuous process or the like.

In the case of a batch process, examples thereof include a method of putting the crude 3,5,5-trimethylhexanoic acid and water into a mixing tank, stirring preferably for 10 seconds to two hours, then leaving still preferably for one minute to two hours for phase separation and obtaining the organic layer containing 3,5,5-trimethylhexanoic acid. An operation of further adding water to the obtained organic layer containing 3,5,5-trimethylhexanoic acid and obtaining the organic layer containing 3,5,5-trimethylhexanoic acid after phase separation may be repeated, and the operation is repeated preferably once to three times. In this case, the addition amount of the water used is preferably 10 to 300 parts by mass based on 100 parts by mass of the crude 3,5,5-trimethylhexanoic acid per one operation.

After the mixture solution of the crude 3,5,5-trimethylhexanoic acid and water is stirred and left still, ultrasonic waves may be applied to the mixture solution. When ultrasonic waves are applied, the time required for phase separation can be shortened.

As the apparatus for conducting by a continuous process, an apparatus which is generally used for continuous extraction or the like, such as a combination of a mixer and a settler, a spray tower, a packed tower and a tray tower, can be used, but in particular, a packed tower or a tray tower having a theoretical tray number of three or more is preferably used.

### (Bubbling Step)

The bubbling step is a step of bubbling the 3,5,5-trimethylhexanoic acid obtained in the water washing step with an inert gas.

The organic layer containing 3,5,5-trimethylhexanoic acid obtained in the water washing step is bubbled with an inert gas to remove water and t-BuOH.

As the inert gas, for example, nitrogen gas can be used.

The amount of the inert gas introduced for bubbling is preferably 0.5 to 15 1/min, more preferably 2.5 to 7.5 1/min per 1 kg of the organic layer. The temperature of the bubbling step is 5°C or higher and lower than 40°C, and the bubbling time is generally one to 30 hours, preferably five to 15 hours.

### <Method for Improving Storage Stability of 3,5,5-Trimethylhexanoic Acid Composition>

The method for improving storage stability of a 3,5,5-trimethylhexanoic acid composition according to the embodiment is a method for improving storage stability of a 3,5,5-trimethylhexanoic acid composition containing 3,5,5-trimethylhexanoic acid and t-butyl alcohol, and
the t-butyl alcohol concentration in terms of toluene-d8 measured by gas chromatography-olfactometry/mass spectrometry under the conditions above is adjusted to exceed 0 ppb by volume and to be 100 ppb by volume or less.

The "method for improving storage stability of a 3,5,5-trimethylhexanoic acid composition according to the embodiment" is sometimes simply referred to as "the method for improving storage stability according to the embodiment" below.

In the method for improving storage stability according to the embodiment, the t-butyl alcohol concentration in terms of toluene-d8 is preferably adjusted to 95 ppb by volume or less, more preferably 70 ppb by volume or less, further preferably 40 ppb by volume or less.

The storage stability of the 3,5,5-trimethylhexanoic acid composition means the capability of preventing t-BuOH from easily increasing and of continuously keeping the reproductive toxicity low even after long-term storage of the 3,5,5-trimethylhexanoic acid composition, and the capability can be improved by the method for improving storage stability of a 3,5,5-trimethylhexanoic acid composition according to the embodiment.

Specifically, the t-butyl alcohol concentration in terms of toluene-d8 measured by gas chromatography-olfactometry/mass spectrometry after heating in an air atmosphere at 80°C for four weeks under the conditions above can be adjusted to 400 ppb by volume or less.

"After heating in an air atmosphere at 80°C for four weeks" means accelerated conditions for creating a state in which the 3,5,5-trimethylhexanoic acid composition is stored for a long time.

The measurement conditions of the gas chromatography-olfactometry/mass spectrometry are the same for both the t-butyl alcohol concentration in terms of toluene-d8 without the "heating in an air atmosphere at 80°C for four weeks" and the t-butyl alcohol concentration in terms of toluene-d8 after heating in an air atmosphere at 80°C for four weeks.

### [t-Butyl Alcohol Concentration in Terms of Toluene-d8]

According to the method for improving storage stability according to the embodiment, the t-BuOH concentration of the 3,5,5-trimethylhexanoic acid composition can be adjusted to 400 ppb by volume or less even after long-term storage of the 3,5,5-trimethylhexanoic acid composition, and the t-BuOH concentration after heating in an air atmosphere at 80°C for four weeks is preferably 370 ppb by volume or less, more preferably 340 ppb by volume or less, further preferably 200 ppb by volume or less.

### [Examples]

Next, the technique of the present disclosure will be specifically explained referring to Examples, but the technique of the present disclosure is not restricted by these examples.

### [Example 1]

### (Synthesis Step)

Crude 3,5,5-trimethylhexanoic acid was synthesized referring to the method described in WO2022118917A1. That is, 3,5,5-trimethylhexanal was synthesized by hydroformylation reaction of diisobutylene and oxo gas, and crude 3,5,5-trimethylhexanoic acid was obtained by subsequent oxidation reaction.

### (Distillation Step)

Next, 239.50 g of the crude 3,5,5-trimethylhexanoic acid was put into a 300-mL three-neck flask having a reflux condenser and a thermometer and distilled at a reduced pressure of 25 kPa, and the fraction of the temperature at the top of the tower of 188 to 191°C was collected. Thus, 184.64 g of a fraction without low-boiling-point components was obtained with a recovery rate of 77.1%.

### (Water Washing Step)

Next, 185 g of the 3,5,5-trimethylhexanoic acid after the distillation step and 370 g of water were put into a 1-L glass sample bottle, stirred by shaking at room temperature for one minute, then irradiated with ultrasonic waves at a temperature of 20°C for one minute using an ultrasonic cleaner manufactured by Sharp Manufacturing Systems Corporation (UT-206H) and then left still for 30 minutes to separate the phases into an aqueous layer and an organic layer.

### (Bubbling Step)

Next, the organic layer after the water washing step was put into a short-neck flask, and by introducing nitrogen at a flow rate of 800 mL/minute through a sparger at room temperature and stirring for 10 hours, 183 g of a 3,5,5-trimethylhexanoic acid composition was obtained.

As a result of gas chromatography mass spectrometry with a concentrator of the obtained 3,5,5-trimethylhexanoic acid composition under the conditions described below, the t-butyl alcohol concentration in terms of toluene-d8 was 93 ppb by volume.

### [Storage Stability Test]

The 3,5,5-trimethylhexanoic acid composition in an amount of 10 g was put into a 20-mL glass container in an air atmosphere, and the container was closed tightly and left still in a thermostat which was kept at 80°C. After four weeks of leaving still in the thermostat, the 3,5,5-trimethylhexanoic acid composition was taken out.

As a result of gas chromatography mass spectrometry with a concentrator under the conditions described below of the 3,5,5-trimethylhexanoic acid composition taken out after the storage stability test, the t-butyl alcohol concentration in terms of toluene-d8 was 362 ppb by volume.

### [Example 2]

The same procedures as those in Example 1 were conducted except that the water washing step was conducted twice. As a result of gas chromatography mass spectrometry with a concentrator of the obtained 3,5,5-trimethylhexanoic acid composition under the conditions described below, the t-butyl alcohol concentration in terms of toluene-d8 was 35 ppb by volume.

As a result of gas chromatography mass spectrometry with a concentrator under the conditions described below of the 3,5,5-trimethylhexanoic acid composition after the storage stability test, the t-butyl alcohol concentration in terms of toluene-d8 was 337 ppb by volume.

### [Example 3]

The same procedures as those in Example 1 were conducted except that the water washing step was conducted three times. As a result of gas chromatography mass spectrometry with a concentrator of the obtained 3,5,5-trimethylhexanoic acid composition under the conditions described below, the t-butyl alcohol concentration in terms of toluene-d8 was 27 ppb by volume.

As a result of gas chromatography mass spectrometry with a concentrator under the conditions described below of the 3,5,5-trimethylhexanoic acid composition after the storage stability test, the t-butyl alcohol concentration in terms of toluene-d8 was 153 ppb by volume.

### [Comparative Example 1]

The same procedures as those in Example 1 were conducted except that the water washing step and the bubbling step were not conducted. As a result of gas chromatography mass spectrometry with a concentrator of the obtained 3,5,5-trimethylhexanoic acid composition under the conditions described below, the t-butyl alcohol concentration in terms of toluene-d8 was 213 ppb by volume.

As a result of gas chromatography mass spectrometry with a concentrator under the conditions described below of the 3,5,5-trimethylhexanoic acid composition after the storage stability test, the t-butyl alcohol concentration in terms of toluene-d8 was 529 ppb by volume.

### <Gas Chromatography-Olfactometry/Mass Spectrometry>

The gas chromatography-olfactometry/mass spectrometry of the 3,5,5-trimethylhexanoic acid compositions before and after the storage stability test in Examples 1 to 3 and Comparative Example 1 was conducted.

### (Concentration of Sample)

First, each 3,5,5-trimethylhexanoic acid composition obtained was concentrated using the concentrator below under the conditions below.
Concentrator: Entech7200 automated concentrator manufactured by ENTECH INSTRUMENTS
Sample amount: 5.0 g
Container volume: 500 mL
Injection amount: 200 mL of the gas phase in the container left still at 30°C for 20 minutes or more
Internal standard substance: 100 mL (standard toluene-d8 gas: concentration of 10 ppb by volume, nitrogen as a dilution gas, Sumitomo Seika Chemicals Company, Limited.)
Concentration method: CTD mode (Cold Trap Dehydration)
Temperature condition of Dehydration Module 1 (Empty Trap: a ceramic-coated trap without adsorbent filling): Trap Temp. -40°C, Desorption Temp. 0°C
Temperature condition of Cold Tenax (registered trademark) Module 2 (Tenax TA Trap: a ceramic-coated trap filled with Tenax TA as an adsorbent): Trap Temp. -30°C, Desorption Temp. 200°C
Temperature condition of Focusing Module 3 (Cryo focusing): Trap Temp. -165°C, Desorption Temp. 100°C
Sample flow rate: 50 mL/min
He Flush Volume: 75 mL
M1 to M2 Volume: 40 mL (100 mL/min)
M2 to M3 Time: 3.0 min
Injection time: 0.3 min

Here, Tenax TA is low polarity porous polymer beads using 2,6-diphenyl-p-phenylene oxide as a base.

Next, gas chromatography-olfactometry/mass spectrometry of the concentrated sample was conducted using the gas chromatography-olfactometry (GC-olfactometry) apparatus and the mass spectrometry detector (MSD) below.

### (GC-Olfactometry)

Measurement device: Agilent 7890B gas chromatography system manufactured by Agilent Technologies
Analysis column: DB-1 manufactured by Agilent Technologies (a column using dimethylpolysiloxane as a stationary phase; a length of 60 m × an inner diameter of 320 µm × a film thickness of 1 µm)
Heating program: After maintaining at 35°C for two minutes, the temperature is increased at 10°C/minute, and the temperature is maintained for seven minutes and 30 seconds after the temperature reaches 240°C.
Carrier gas: helium
Control mode: constant pressure (153.09 kPa)

The concentrated sample is separated by a capillary column and then sent to ODP3 (sniffing port) and MSD5977B at a ratio of 1/1.

### (MSD)

Detector: Agilent 5977B MSD manufactured by Agilent Technologies
Ionization mode: EI
Measurement type: scan
Ion source temperature: 250°C
Quadrupole temperature: 150°C
Electron energy: 70.0 eV
Initial mass of scanning: 30
Final mass of scanning: 400
Calibration curve: created using standard toluene-d8 gas manufactured by Sumitomo Seika Chemicals Company, Limited. (concentration: 10 ppb by volume, dilution gas: nitrogen).

### (Data Analysis)

For the data analysis, an extracted ion chromatogram (EIC) was used, and the EIC peak area of t-butyl alcohol (EIC: m/z 59.000) that appeared in the relative retention time of 0.48 to 0.56 was observed, where the relative retention time of toluene-d8 was regarded as 1.0. The t-butyl alcohol concentration in terms of toluene-d8 of the 3,5,5-trimethylhexanoic acid vapor was calculated using an equation derived from the calibration curve, "concentration in terms of toluene-d8 (ppb by volume) = 3.94 × 10⁻⁶ × EIC peak area", where the sensitivity of the EIC peak of t-butyl alcohol and the sensitivity of the EIC peak of toluene-d8 were supposed to be equal.

The results are shown in Table 1.

**Table 1**

| | Storage Stability Test | Example 1 | Example 2 | Example 3 | Comparative Example 1 |
|---|---|---|---|---|---|
| t-Butyl alcohol concentration in terms of toluene-d8 (ppb by volume) | Before Test | 93 | 35 | 27 | 213 |
| | After Test | 362 | 337 | 153 | 529 |

The t-butyl alcohol concentrations in terms of toluene-d8 of the 3,5,5-trimethylhexanoic acid compositions obtained in Examples 1 to 3 measured by gas chromatography-olfactometry/mass spectrometry were 400 ppb by volume or less also after the storage stability test. That is, it can be seen that the 3,5,5-trimethylhexanoic acid compositions obtained in the Examples are excellent in suppressing reproductive toxicity even after long-term storage.

### Industrial Applicability

According to the present invention, a 3,5,5-trimethylhexanoic acid composition which has low reproductive toxicity even after long-term storage and a method for improving the storage stability thereof can be provided. The 3,5,5-trimethylhexanoic acid according to the embodiment can be used as a raw material of a lubricating oil (for example, a refrigeration oil), metallic soap, a plasticizer, a surfactant, an alkyd resin, a fatty acid chloride, a metal working oil, a cosmetic product or the like.

## Claims

1. A 3,5,5-trimethylhexanoic acid composition comprising 3,5,5-trimethylhexanoic acid and t-butyl alcohol,
wherein the t-butyl alcohol concentration in terms of toluene-d8 measured by gas chromatography-olfactometry/mass spectrometry after heating in an air atmosphere at 80°C for four weeks under the conditions below exceeds 0 ppb by volume and is 400 ppb by volume or less:
(Measurement Conditions)
· the 3,5,5-trimethylhexanoic acid composition in an amount of 5.0 g is filled into a sealable 500-mL container and left still at 30°C for 20 minutes or more. Then, 200 mL of the gas phase is sucked, and the residue after removing H₂O, N₂, O₂ and CO₂ is concentrated with a concentrator, and
· the concentrated sample is measured by gas chromatography-olfactometry under the conditions below, and the structure of a measured component is analyzed with a mass spectrometry detector:
Analysis column: a column using dimethylpolysiloxane as a stationary phase; a length of 60 m × an inner diameter of 320 µm × a film thickness of 1 µm
Heating program: After maintaining at 35°C for two minutes, the temperature is increased at 10°C/minute, and the temperature is maintained for seven minutes and 30 seconds after the temperature reaches 240°C.
Carrier gas: helium
The measurement conditions of the mass spectrometry detector are as follows.
Ionization mode: EI
Measurement type: scan
Ion source temperature: 250°C
Quadrupole temperature: 150°C
Electron energy: 70.0 eV
Initial mass of scanning: 30
Final mass of scanning: 400.

2. The 3,5,5-trimethylhexanoic acid composition according to claim 1 which is used for a cosmetic product.

3. The 3,5,5-trimethylhexanoic acid composition according to claim 1 which is used for a refrigeration oil.

4. A method for improving storage stability of a 3,5,5-trimethylhexanoic acid composition containing 3,5,5-trimethylhexanoic acid and t-butyl alcohol,
wherein the t-butyl alcohol concentration in terms of toluene-d8 measured by gas chromatography-olfactometry/mass spectrometry under the conditions below is adjusted to exceed 0 ppb by volume and to be 100 ppb by volume or less:
(Measurement Conditions)
· the 3,5,5-trimethylhexanoic acid composition in an amount of 5.0 g is filled into a sealable 500-mL container and left still at 30°C for 20 minutes or more. Then, 200 mL of the gas phase is sucked, and the residue after removing H₂O, N₂, O₂ and CO₂ is concentrated with a concentrator, and
· the concentrated sample is measured by gas chromatography-olfactometry under the conditions below, and the structure of a measured component is analyzed with a mass spectrometry detector:
Analysis column: a column using dimethylpolysiloxane as a stationary phase; a length of 60 m × an inner diameter of 320 µm × a film thickness of 1 µm
Heating program: After maintaining at 35°C for two minutes, the temperature is increased at 10°C/minute, and the temperature is maintained for seven minutes and 30 seconds after the temperature reaches 240°C.
Carrier gas: helium
The measurement conditions of the mass spectrometry detector are as follows.
Ionization mode: EI
Measurement type: scan
Ion source temperature: 250°C
Quadrupole temperature: 150°C
Electron energy: 70.0 eV
Initial mass of scanning: 30
Final mass of scanning: 400.

5. The method for improving storage stability of a 3,5,5-trimethylhexanoic acid composition according to claim 4,
which is a method for improving the storage stability of the 3,5,5-trimethylhexanoic acid composition containing 3,5,5-trimethylhexanoic acid and t-butyl alcohol,
wherein the t-butyl alcohol concentration in terms of toluene-d8 measured by gas chromatography-olfactometry/mass spectrometry after heating in an air atmosphere at 80°C for four weeks under the conditions is adjusted to exceed 0 ppb by volume and to be 400 ppb by volume or less by adjusting the t-butyl alcohol concentration in terms of toluene-d8 measured by gas chromatography-olfactometry/mass spectrometry under the conditions to exceed 0 ppb by volume and to be 100 ppb by volume or less.
